# EUROPÄISCHE PATENTSCHRIFT

(11) **EP 2 012 747 B1**
(45) Veröffentlichungstag und Bekanntmachung des Hinweises auf die Patenterteilung: **21.07.2010**
(21) Anmeldenummer: 07724185.9
(22) Anmeldetag: 12.04.2007
(51) Int. Cl.: A61K 9/00, C08G 18/08, C08G 18/12, A23G 4/00

(54) **VERWENDUNG VON WÄSSRIGEN POLYURETHAN-DISPERSIONEN ALS BASIS FÜR KAUMASSEN IN KOSMETISCHEN PRODUKTEN**
USE OF AQUEOUS POLYURETHANE DISPERSIONS FOR MASTICATORY MASSES IN COSMETIC PRODUCTS
UTILISATION DE DISPERSIONS D'POLYURETHANE AQUEOUSES POUR PRODUITS À MÂCHER EN PRODUITS COSMÉTIQUES

(30) Priorität: 22.04.2006 DE 102006018826; 26.04.2006 DE 102006019742
(43) Veröffentlichungstag der Anmeldung: 14.01.2009
(73) Patentinhaber: Bayer MaterialScience AG, 51368 Leverkusen (DE)
(72) Erfinder: NIESTEN, Meike, 51061 Köln (DE); HOFACKER, Steffen, 51519 Odenthal (DE); RISCHE, Thorsten, 59423 Unna (DE); DÖRR, Sebastian, 40597 Düsseldorf (DE); FELLER, Thomas, 42659 Solingen (DE); MICHAELIS, Thomas, 51375 Leverkusen (DE)
(86) Internationale Anmeldenummer: PCT/EP2007/003244
(87) Internationale Veröffentlichungsnummer: WO 2007/121867

(56) Entgegenhaltungen:
- WO-A-98/31297
- WO-A-02/090413
- WO-A-2004/096886
- US-A- 4 149 815

## Beschreibung

Die Erfindung betrifftt die Verwendung von Kaumassen für den Mundpflegebereich auf Basis wässriger Polyurethandispersionen geschäumter.

Organische Polymere sind als Rohstoffe in kosmetischen Produkten weit verbreitet. Sie sind in vielerlei kosmetischer Erzeugnisse wie z.B. in Haarsprays, Haargels, Mascara, Lippenstifte, Cremes etc. zu finden. Im Bereich der Mundpflege (Oral Care) sind Polymere z.B. in Form von Zahnbürsten, Zahnseiden etc. zu finden.

Aufgrund des aufkommenden Bedürfnisses der Gesellschaft zur Mundpflege für die Zeiträume zwischen den Mahlzeiten bzw. nach Genuss z.B. einer Zwischenmahlzeit oder anderer Genussmittel (wie. z.B. Süßigkeiten, Nikotin, Alkohol, etc.) oder aber auch aufgrund der erhöhten Mobilität (z.B. auf Flug- oder Bahnreisen) in denen eine gewöhnliche Zahnreinigung mit Wasser, Zahncreme und Zahnbürste nicht möglich ist, sind in der Vergangenheit Produkte wie Zahnpflegekaugummis oder auch Zahnpflegetücher entwickelt worden.

Zahnpflegekaugummis bestehen im Wesentlichen aus sog. Kaugummi-Base. Diese wiederum besteht aus natürlichen oder künstlichen Polymeren wie z.B. Latex, Polyvinylether, Polyisobutylenvinylether, Polyisobuten, etc.. Derartige Zahnpflegekaugummis enthalten als zahnpflegende Mittel in der Regel pH-Wert kontrollierende Substanzen, die somit der Entstehung von Zahnfäule (Karies) entgegenwirken. Aufgrund ihres plastischen Verhaltens, tragen derartige Zahnpflegekaugummis jedoch kaum zu Reinigung der Kauflächen bzw. Zahnseiten bei. Ferner weisen Kaugummis generell den Nachteil auf, dass sie oftmals von öffentlichen Straßen und Plätzen mechanisch entfernt und entsorgt werden müssen, was zu erheblichen Reinigungsaufwand - aufgrund ihrer klebenden Eigenschaften - der Boden- und Straßenbeläge führt.

Zahnpflegetücher (z.B. Oral-B Brush Aways™, Gillette GmbH & Co. OHG, Deutschland) zeichnen sich dadurch aus, dass sie durch Aufbringung des Zahnpflegetuches auf einen Finger und durch Abreiben der Zähne eine gute Reinigungswirkung der Zahnseiten erreichen. Allerdings ist die Art der Anwendung derartiger Zahnreinigungstücher in der Öffentlichkeit aus ästetischen Gründen wenig akzeptiert und stellt somit keine Alternative zur Benutzung einer herkömmlichen Zahnbürste dar.

Es wurde nun gefunden, dass sich aus Wässrigen Polyurethan Dispersionen geschäumte Materialien herstellen lassen, welche sich unter anderem aufgrund ihrer besonders vorteilhaften mechanischen Eigenschaften als Kaumassen für den Mundpflegebereich eignen.

Gegenstand der Erfindung ist daher die Verwendung Kaumassen aus geschäumten wässrigen Polyurethan-Dispersionenen.

Eine erfindungswesentliche Eigenschaft der verwendeten Kaumassen besteht darin, dass sie während des Kauens eine Formstabilität aufweisen, d.h. keine plastische Verformung wie beispielsweise Kaugummis aus dem Stand der Technik erleiden, sondern vielmehr nach Belastung in einem Kauvorgang in ihre ursprüngliche Form aufgrund der vorhandenn polymeren Rückstellkräfte zurückkehren. Erst dann ist gewährleistet, dass auch eine zahnreinigende Wirkung (vor allem auch der Zahnseiten) eintreten kann.

Bevorzugt ist es, wenn die erfindungsgemäßen Kaumassen ein Zugmodul bei 100 % Dehnung von 0,1 bis 8,0 MPa, bei einer Zugfestigkeit von 0,5 bis 80 MPa und einer Dehnbarkeit von 100 bis 3000 % aufweisen.

Besonders bevorzugt sind solche, die ein Zugmodul bei 100% Dehnung von 0,3 bis 3,5 MPa, bei einer Zugfestigkeit von 0,5 bis 40 MPa und einer Dehnbarkeit von 200 bis 2000 % aufweisen.

Die Zugversuche wurde durchgeführt gemäß DIN 53504 mit einem Probekörper Schulterstab S2 gemäß DIN 53504. Die Zugmodule wurden gemäß DIN EN ISO 527 ermittelt. Die Schichtdicke der Probekörper betrug 2,5 mm +/- 1 mm).

Ferner ist es vorteilhaft, wenn das Verhältnis aus Zugfestigkeit und Elastizitätsmodul der erfindungsgemäßen polymeren Kaumasse größer gleich 1, bevorzugt größer 1,5 und besonders bevorzugt größer 2 ist und das Verhältnis aus dem Produkt der Weiterreissfestigkeit (gemäß DIN ISO 34-1 (2004)) und Elastizitätsmodul zum Quadrat der Zugfestigkeit kleiner als 4 mm, bevorzugt kleiner als 1,5 mm ist.

Darüber hinaus sollte die Stabilität der polymeren Kaumasse unter Kompression größer als 50 MPa, bevorzugt größer 75 MPa sein.

Wässrige Polyurethan-Dispersionen lassen sich unter Zuhilfenahme von Treibgasen oder mechanischer Energie aufschäumen. Dabei kann es von Vorteil sein, wenn man Schaumhilfsmittel zusetzt, um eine stabile Schaumstruktur zu erhalten.

Die Verwendeten Kaumasen basier auf wässrige Polyurethan-Dispersionen.

Grundsätze zur Herstellung von Schäumen sind beispielsweise in *"*Fundamentals of Foam Formation" (J.H. Saunders, Chapter 2, Polymer Foams, Editors Klempner, Frisch, Carl Hanser Verlag Munich, 1991) beschrieben.

Um die wässrigen Polyurethan-Dispersionenen erfindungsgemäß schäumen zu können, werden diese bevorzugt zunächst als Flüssigphase bereitgestellt. Falls die Bestandteile der Schäume nicht per se als Flüssigkeit vorliegen, kann dies durch Lösen nicht flüssiger Bestandteile in einer flüssigen Komponente erfolgen. Ebenfalls möglich ist hierzu der Einsatz von organischen Lösungsmitteln, Weichmachern, Wasser oder das Aufschmelzen, um die Bestandteile in bei Schäumungsbedingungen flüssiger Phase beispielsweise als Lösung, Dispersion oder Schmelze bereitzustellen.

Das eigentliche Aufschäumen erfolgt durch Eintrag von Luft, Stickstoff-Gas, niedrig siedender Flüssigkeiten wie Pentan, Fluorkohlenwasserstoffe, Methylenchlorid.

Eine Aushärtung im Anschluss an die Schaumbildung erfolgt bei Verwendung wässriger Polyurethandispersionen, die zunächst aufgeschäumt und danach erst getrocknet werden. Aushärtung kann neben chemischer Vernetzung oder physikalischer Trocknung auch durch Temperaturabsenkung einer Schmelze, Gelieren von Plastisolen oder Koagulation beispielsweise von Latices erfolgen.

"Aushärtung unter Erhalt der Schaumstruktur" meint im Rahmen der vorliegenden Erfindung, dass die aufgeschäumte Mischung so in den festen Zustand überführt wird, dass es nicht zu einem Kollabieren des Schaumes unter Verlust der zelligen Struktur des Schaumes kommt. Hierbei werden dann Schäume erhalten, die in einer bevorzugten Ausführungsform die weiter unten genannten Schaumdichten aufweisen.

Aushärtung durch physikalische Trocknung erfolgt bevorzugt bei einer Temperatur von 25 bis 150°C, bevorzugt 30°C bis 120°C, besonders bevorzugt bei 40 bis 100°C. Die Trocknung kann in einem konventionellen Trockner erfolgen.

Bei der Herstellung der erfindungsgemäßen Kaumassen können neben synthetischen oder chemisch modifizierten natürlichen Polymeren oder den zu ihrer Bildung notwendigen Ausgangsstoffe (I) auch Schaumhilfsmittel (II), Vernetzer (III), Verdicker (IV), Hilfsmittel (V) und kosmetische Zusatzstoffe (VI) mitverwendet werden.

Als Schaumhilfsmittel (II) sind handelsübliche Schaumgeneratoren und/oder -stabilisatoren geeignet, wie wasserlösliche Fettsäureamide, Sulfosuccinamide, Kohlenwasserstoffsulfonate, sulfate oder Fettsäuresalze, wobei der lipophile Rest bevorzugt 12 bis 24 Kohlenstoffatome enthält.

Bevorzugte Schaumhilfsmittel (II) sind Alkansulfonate oder -sulfate mit 12 bis 22 Kohlenstoffatomen im Kohlenwasserstoffrest, Alkylbenzosulfonate oder -sulfate mit 14 bis 24 Kohlenstoffatomen im Kohlenwasserstoffrest oder Fettsäureamide oder Fettsäuresalze mit 12 bis 24 Kohlenstoffatomen.

Bei vorgenannten Fettsäureamiden handelt es sich vorzugsweise um Fettsäureamide von Mono- oder Di-(C2-3-alkanol)-aminen. Fettsäuresalze können beispielsweise Alkalimetallsalze, Aminsalze oder unsubstituierte Ammoniumsalze sein.

Solche Fettsäurederivate basieren typischerweise auf Fettsäuren wie Laurinsäure, Myristinsäure, Palmitinsäure, Ölsäure, Stearinsäure, Ricinolsäure, Behensäure oder Arachidinsäure, Kokosfettsäure, Talgfettsäure, Sojafettsäure und deren Hydrierungsprodukten.

Besonders bevorzugte Schaumhilfsnüttel (II) sind Natriumlaurylsulfat, Sulfosuccinamide und Ammoniumstearate, sowie Mischungen daraus.

Geeignete Vernetzer (III) sind beispielsweise unblockierte Polyisocyanat-Vernetzer, Amid- und Amin-Formaldehydharze, Phenolharze, Aldehyd- und Ketonharze, wie z.B. Phenol-Formaldehydharze, Resole, Furanharze, Harnstoffharze, Carbamidsäureesterharze, Triazinharze, Melaminharze, Benzoguanaminharze, Cyanamidharze oder Anilinharze.

In einer besonders bevorzugten Ausführungsform wird auf den Einsatz von Vernetzern (III) vollständig verzichtet.

Verdicker (IV) im Sinne der Erfindung sind Verbindungen, die es ermöglichen, die Viskosität der Bestandteile bzw. ihrer Mischungen so einzustellen, dass die Erzeugung und Verarbeitung des erfindungsgemäßen Schaumes begünstigt. Als Verdicker sind handelsübliche Verdicker geeignet, wie beispielsweise natürliche organische Verdicker, z.B. Dex-trine oder Stärke, organisch abgewandelte Naturstoffe, z.B. Celluloseether oder Hydroxy-ethylcellulose, organisch vollsynthetische, z.B. Polyacrylsäuren, Polyvinylpyrrolidone, Poly(meth)acrylverbindungen oder Polyurethane (assoziative Verdicker) sowie anorganische Verdicker, z.B. Betonite oder Kieselsäuren. Bevorzugt werden organisch vollsynthetische Verdicker eingesetzt. Besonders bevorzugt werden Acrylatverdicker verwendet, die vor der Zugabe gegebenenfalls weiter mit Wasser verdünnt werden. Bevorzugte handelsübliche Verdicker sind beispielsweise Mirox^{®} AM (BGB Stockhausen GmbH, Krefeld, Deutschland), Walocel^{®} MT 6000 PV (Wolff Cellulosics GmbH & Co KG, Walsrode, Deutschland), Rheolate^{®} 255 (Elementies Specialities, Gent, Belgien), Collacral^{®} VL (BASF AG, Ludwigshafen, Deutschland) und Aristoflex^{®} AVL (Clariant GmbH Sulzbach, DE).

Hilfsmittel (V) im Sinne der Erfindung sind z.B. Antioxidantien und/oder Lichtschutzmittel und/oder andere Zusatzmittel wie beispielsweise Emulgatoren, Füllstoffe, Weichmacher, Pigmente, Kieselsäuresole, Aluminium-, Ton-, Dispersionen, Verlaufsmittel oder Thixotropiemittel, etc..

Kosmetische Zusatzstoffe (VI) im Sinne der Erfindung sind z.B. Geschmack- und Aromastoffe, Abrasivstoffe, Farbstoffe, Süßmittel, etc. sowie aktive Ingredienzien, wie Fluorid-Verbindungen, Zahnweißmacher etc.

Schaumhilfsmittel (II), Vernetzer (III), Verdicker (IV) und Hilfsmittel (V) können jeweils bis zu 20 Gew.-% und kosmetische Zusatzstoffe (VI) bis zu 80 Gew.-% bezogen auf die geschäumte und getrocknete Kaumasse ausmachen.

Bevorzugt werden im erfindungsgemäßen Verfahren 80 bis 99,5 Gew.-% der synthetischen oder chemisch modifizierten natürlichen Polymeren oder der zu ihrer Bildung notwendigen Ausgangsstoffe (I), 0 bis 10 Gew.-% der Komponente (II), 0 bis 10 Gew.-% der Komponente (III), 0 bis 10 Gew.-% der Komponente (IV), 0 bis 10 Gew.-% der Komponente (V) und 0,1 bis 20 Gew.-% der Komponente (VI) eingesetzt, wobei sich die Summe auf die nichtflüchtigen Anteile der Komponenten (I) bis (VI) bezieht und sich die Summe der Einzelkomponenten (I) bis (VI) zu 100 Gew.-% addiert.

Besonders bevorzugt werden im erfindungsgemäßen Verfahren 80 bis 99,5 Gew.-% der synthetischen oder chemisch modifizierten natürlichen Polymeren oder der zu ihrer Bildung notwendigen Ausgangsstoffe (I), 0 bis 10 Gew.-% der Komponente (II), 0 bis 10 Gew.-% der Komponente (IV), 0 bis 10 Gew.-% der Komponente (V) und 0,1 bis 15 Gew.-% der Komponente (VI) eingesetzt, wobei sich die Summe auf die nichtflüchtigen Anteile der Komponenten (I) bis (VI) bezieht und sich die Summe der Einzelkomponenten (I) bis (VI) zu 100 Gew.-% addiert.

Ganz besonders bevorzugt sind 80 bis 99,5 Gew.-% der synthetischen oder chemisch modifizierten natürlichen Polymeren oder der zu ihrer Bildung notwendigen Ausgangsstoffe (I), 0,1 bis 10 Gew.-% der Komponente (II), 0,1 bis 10 Gew.-% der Komponente (IV), 0,1 bis 10 Gew.-% der Komponente (V) und 0,1 bis 15 Gew.-% der Komponente (VI), wobei sich die Summe auf die nichtflüchtigen Anteile der Komponenten (I) bis (VI) bezieht und sich die Summe der Einzelkomponenten (I) bis (VI) zu 100 Gew.-% addiert.

Die geschäumte Zusammensetzung kann auf verschiedenste Arten und Weisen auf verschiedenste Oberflächen oder in Formen appliziert werden. Bevorzugt ist jedoch Gießen, Rakeln, Walzen, Streichen, Spritzen oder Sprühen.

Zur Formgebung kann die aufzuschäumende oder bereits aufgeschäumte Mischung zunächst auf eine Oberfläche oder in eine Form gegeben werden, bevor sie weiterverarbeitet wird.

Während das geschäumte Material vor Aushärtung eine bevorzugte Schaumdichte von 200 bis 800 g/l, besonders bevorzugt 200 bis 700 g/l, ganz besonders bevorzugt 300 bis 600 g/l aufweist, beträgt die Dichte der resultierenden erfindungsgemäßen Kaumasse nach der Trocknung vorzugsweise 50 bis 600 g/l, besonders bevorzugt 100 bis 500 g/l.

Did Kaumassen haben nach dem Trocknungsschritt typischerweise eine Dicke von 1 mm bis 100 mm, 1 mm bis 50 mm, bevorzugt 1 mm bis 30 mm.

Die Kaumassen können auch in mehreren Schichten beispielsweise zur Erzeugung besonders hoher Schaumauflagen, auf die verschiedensten Substrate aufgetragen werden oder in Formen gegossen werden.

Darüber hinaus können die Verwendeten geshäumten Zusammensetzungen auch in Kombination mit anderen Trägermaterialien wie z.B. textile Träger, Papier etc. eingesetzt werden, beispielsweise durch vorheriges Auftragen (z.B. Beschichten).

Die Verwendeten Kaumassen besitzen exzellente mechanische Eigenschaften, insbesondere eine hohe Dehnbarkeit bei hoher Zugfestigkeit; Kehren somit nach dem Kauvorgang in ihre Ursprungsform zurück, besitzen die Fähigkeit die Kauflächen und Zahnseiten zu reinigen und kleben nicht auf Bodenbelägen.

In der Erfindung werden Polyurethane in Form wässriger Dispersionen (I) eingesetzt.

Solche Polyurethan-Polyharnstoff-Dispersionen (I) sind erhältlich, in dem
A) isocyanatfunktionelle Prepolymere aus
   a1) organischen Polyisocyanaten
   a2) polymeren Polyolen mit zahlenmittleren Molekulargewichten von 400 bis 8000 g/mol und OH-Funktionalitäten von 1,5 bis 6,
   a3) gegebenenfalls hydroxyfunktionellen Verbindungen mit Molekulargewichten von 62 bis 399 g/mol und
   a4) gegebenenfalls hydroxyfunktionellen, ionischen oder potentiell ionischen und/oder nichtionischen Hydrophilierungsmitteln,
   hergestellt werden,
B) deren freie NCO-Gruppen dann ganz oder teilweise mit
   b1) aminofunktionellen Verbindungen mit Molekulargewichten von 32 bis 400 g/mol und/oder
   b2) aminofunktionellen, ionischen oder potentiell ionischen Hydrophilierungsmitteln
unter Kettenverlängerung umgesetzt werden und die Prepolymere vor während oder nach Schritt B) in Wasser dispergiert werden, wobei gegebenenfalls enthaltene potentiell ionische Gruppen durch teilweise oder vollständige Umsetzung mit einem Neutralisationsmittel in die ionische Form überführt werden können.

Isocyanatreaktive Gruppen sind beispielsweise Amino-, Hydroxy- oder Thiolgruppen.

Beispiele solcher in Komponente a1) einsetzbaren organischen Polyisocyanate sind 1,4-Butylendiisocyanat, 1,6-Hexamethylendiisocyanat (HDI), Isophorondiisocyanat (IPDI), 2,2,4 und/oder 2,4,4-Trimethylhexamethylendiisocyanat, die isomeren Bis-(4,4'-isocyanatocyclohexyl)methane oder deren Mischungen beliebigen Isomerengehalts, 1,4-Cyclohexylendiisocyanat, 1,4-Phenylendiisocyanat, 2,4- und/oder 2,6-Toluylendüsocyanat, 1,5-Naphthylendiisocyanat, 2,2'-und/oder 2,4'-und/oder 4,4'-Diphenylmethandiisocyanat, 1,3- und/oder 1,4-Bis-(2-isocyanato-prop-2-yl)-benzol (TMXDI), 1,3-Bis(isocyanato-methyl)benzol (XDI), (S)-alkyl 2,6-diisocyanatohexanoate, (L)-alkyl 2,6-diisocyanatohexanoate, mit verzweigten, cyclischen oder acyclischen Alkylgruppen mit bis zu 8 C-Atomen.

Neben den vorstehend genannten Polyisocyanaten können anteilig auch modifizierte Diisocyanate mit Uretdion-, Isocyanurat-, Urethan-, Allophanat-, Biuret-, Iminooxadiazindion- und/oder Oxadiazintrionstruktur sowie nicht-modifiziertes Polyisocyanat mit mehr als 2 NCO-Gruppen pro Molekül sei z.B. 4-Isocyanatomethyl-1,8-octandiisocyanat (Nonantriisocyanat) oder Triphenylmethan-4,4',4"-triisocyanat mit eingesetzt werden.

Bevorzugt handelt es sich um Polyisocyanate oder Polyisocyanatgemische der vorstehend genannten Art mit ausschließlich aliphatisch und/oder cycloaliphatisch gebundenen Isocyanatgruppen und einer mittleren NCO-Funktionalität der Mischung von 2 bis 4, bevorzugt 2 bis 2,6 und besonders bevorzugt 2 bis 2,4.

Besonders bevorzugt werden in a1) 1,6-Hexamethylendiisocyanat, Isophorondiisocyanat, die isomeren Bis-(4,4'-isocyanatocyclohexyl)methane sowie deren Mischungen eingesetzt.

Bevorzugt werden in a2) polymere Polyole mit zahlenmittleren Molekulargewichten von 400 bis 6000 g/mol, besonders bevorzugt von 600 bis 3000 g/mol eingesetzt.

Diese weisen bevorzugt OH-Funktionalitäten von 1,8 bis 3, besonders bevorzugt von 1,9 bis 2,1 1 auf.

Solche polymeren Polyole sind die in der Polywethanlacktechnologie an sich bekannten Polyesterpolyole, Polyacrylatpolyole, Polywethanpolyole, Polycarbonatpolyole, Polyetherpolyole, Polyesterpolyacrylatpolyole, Polywethanpolyacrylatpolyole, Polywethanpolyesterpolyole, Polyurethanpolyetherpolyole, Polyurethanpolycarbonatpolyole und Polyesterpolycarbonatpolyole. Diese können in a2) einzeln oder in beliebigen Mischungen untereinander eingesetzt werden.

Solche Polyesterpolyole sind die an sich bekannten Polykondensate aus Di- sowie gegebenenfalls Tri,- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Neopentylglykol und Hydroxypivalinsäureneopenthylglykolester bevorzugt sind. Daneben können auch Polyole wie Trimethylolpropan, Glycerin, Erythrit, Pentaerythrit, Triemthylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und Phthtalsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können in a2) hydroxylgruppenaufweisende Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-butandiol, 1,6-Hexandiol, 1,8-Octandiol, Neopentylglykol, 1,4-Bishydroxymethylcyclohexan, 2-Methyl-1,3-propandiol, 3-Methyl-1,5-pentandiol, 2,2,4-Trimethylpentandiol-1,3, Dipropylenglykol, Polypropylenglykole, Dibutylenglykol, Polybutylenglykole, Bisphenol A, Tetrabrombisphenol A und lactonmodifizierte Diole der vorstehend genannten Art in Frage. Es können auch Mischungen von verschiedenen Diolen eingesetzt werden.

Bevorzugt enthält die Diolkomponente 40 bis 100 Gew.-% Hexandiol, bevorzugt sind 1,6-Hexandiol und/oder Hexandiolderivate. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole, welche als Diolkomponente neben den beschriebenen Diolen auch Polyetherdiole enthalten, in a2) eingesetzt werden.

Hydroxylgruppenaufweisende Polycarbonate sind bevorzugt linear gebaut, können aber auch durch den Einbau polyfunktioneller Komponenten, insbesondere niedermolekularer Polyole, Verzweigungen enthalten. Hierzu eignen sich beispielsweise Glycerin, Trimethylolpropan, Hexantriol-1,2,6, Butantriol-1,2,4, Trimethylolpropan, Trimethylolethan, Pentaerythrit, Chinit, Mannit, Sorbit, Methylglykosid oder 1,3,4,6-Dianhydrohexite.

Als Polyetherpolyole sind beispielsweise die in der Polyurethanchemie an sich bekannten Polytetramethylenglykolpolyether geeignet, wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxide und/oder Epichlorhydrins an di- oder polyfunktionelle Startermoleküle.

Als geeignete Startermoleküle können alle dem Stand der Technik nach bekannten Verbindungen eingesetzt werden, wie zum Beispiel Wasser, Butyldiglykol, Glycerin, Diethylenglkol, Trimethylolpropan, Propylenglykol, Sorbit, Ethylendiamin, Triethanolamin, 1,4-Butandiol.

Besonders bevorzugte Ausführungsformen der Polyurethan Dispersionen (I) enthalten als Komponente a2) eine Mischung aus Polycarbonatpolyolen und Polytetramethylenglykolpolyolen. Der Anteil der Polycarbonatpolyole in der Mischung beträgt 20 bis 80 Gew.-% und 80 bis 20 Gew.-% an Polytetramethylenglykolpolyolen. Bevorzugt ist ein Anteil von 30 bis 75 Gew.-% an Polytetramethylenglykolpolyolen und 25 bis 70 Gew.-% an Polycarbonatpolyolen. Besonders bevorzugt ist ein Anteil von 35 bis 70 Gew.-% an Polytetramethylenglykolpolyolen und 30 bis 65 Gew.-% an Polycarbonatpolyolen, jeweils mit der Maßgabe, dass die Summe der Gewichtsprozente der Polycarbonat- und Polytetramethylenglykolpolyole 100 Gew.-% ergibt und der Anteil der Summe der Polycarbonat- und Polytetramethylenglykolpolyetherpolyole an der Komponente a2) mindestens 50 Gew.-% bevorzugt 60 Gew.-% und besonders bevorzugt mindestens 70 Gew.-% beträgt.

In a3) können Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit sowie deren beliebige Mischungen untereinander eingesetzt werden.

Geeignet sind auch Esterdiole des genannten Molekulargewichtsbereichs wie α-Hydroxybutyl-εhydroxy-capronsäureester, ω-Hydroxyhexyl-y-hydroxybuttersäure-ester, Adipinsäure-(ß-hydroxyethyl)ester oder Terephthalsäurebis(ß-hydroxyethyl)-ester.

Ferner können in a3) auch monofunktionelle hydroxygruppenhaltige Verbindungen eingesetzt werden. Beispiele solcher monofunktionellen Verbindungen sind Ethanol, n-Butanol, Ethylenglykolmonobutylether, Diethylenglykolmonomethylether, Ethylenglykolmonobutylether, Diethylenglykolmonobutylether, Propylenglykolmonomethylether, Dipropylenglykol-monomethylether, Tripropylenglykolmonomethylether, Dipropylenglykolmono-propylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Unter hydroxyfunktionellen, ionischen oder potentiell ionischen Hydrophilierungsmitteln a4) werden sämtliche Verbindungen verstanden, die mindestens eine isocyanatreaktive Hydroxylgruppe sowie mindestens eine Funktionalität, wie z.B. -COOY, -SO₃Y, -PO(OY)₂ (Y⁺ beispielsweise = H⁺, NH₄⁺, Metallkation), -NR₂, -NR₃⁺ (R = H, Alkyl, Aryl), aufweisen, die bei Wechselwirkung mit wässrigen Medien ein pH-Wert-abhängiges Dissoziationsgleichgewicht eingeht und auf diese Weise negativ, positiv oder neutral geladen sein kann.

Geeignete ionisch oder potentiell ionisch hydrophilierende Verbindungen entsprechend der Definition der Komponente a4) sind z.B. Mono- und Dihydroxycarbonsäuren, Mono- und Dihydroxysulfonsäuren, sowie Mono- und Dihydroxyphosphonsäwen und ihre Salze wie Dimethylolpropionsäure, Dimethylolbuttersäure, Hydroxypivalinsäure, Äpfelsäure, Zitronensäure, Glykolsäure, Milchsäure, das propoxylierte Addukt aus 2-Butendiol und NaHSO₃, z.B. beschrieben in der DE-A 2 446 440 (Seite 5-9, Formel I-III) sowie Verbindungen, die in kationische Gruppen überführbare, z.B. Amin-basierende, Bausteine wie N-Methyl-diethanolamin als hydrophile Aufbaukomponenten enthalten.

Bevorzugte ionische oder potentiell ionische Hydrophilierungsmittel der Komponente a4) sind solche der vorstehend genannten Art, die anionisch, bevorzugt über Carboxy- oder Carboxylat- und/oder Sulfonatgruppen hydrophilierend wirken.

Besonders bevorzugte ionische oder potentiell ionische Hydrophilierungsmittel sind solche, die Carboxyl- und/oder Sulfonatgruppen als anionische oder potentiell anionische Gruppen enthalten, wie die Salze von der Dimethylolpropionsäure oder Dimethylolbuttersäue.

Geeignete nichtionisch hydrophilierende Verbindungen der Komponente a4) sind z.B. Polyoxyalkylenether, die mindestens eine Hydroxy- oder Aminogruppe als isocyanatreaktive Gruppe enthalten.

Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38).

Diese sind entweder reine Polyethylenoxidether oder gemischte Polyalkylenoxidether, wobei sie mindestens 30 mol-%, bevorzugt mindestens 40 mol-% bezogen auf alle enthaltenen Alkylenoxideinheiten an Ethylenoxideinheiten enthalten.

Besonders bevorzugte nichtionische Verbindungen sind monofunktionelle gemischte Polyalkylenoxidpolyether, die 40 bis 100 mol-% Ethylenoxid- und 0 bis 60 mol-% Propylenoxideinheiten aufweisen.

Geeignete Startermoleküle für solche nichtionischen Hydrophilierungsmittel sind gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Als Komponente b1) können Di- oder Polyamine wie 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, 1,3- und 1,4-Xylylendiamin, α,α,α',α'-Tetramethyl-1,3- und -1,4-xylylendiamin und 4,4-Diaminodicyclohexylmethan und/oder Dimethylethylendiamin eingesetzt werden. Ebenfalls möglich ist die Verwendung von Hydrazin oder sowie Hydraziden wie Adipinsäuredihydrazid.

Darüber hinaus können als Komponente b1) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-Methylaminopropan, 3-Amino-1-Ethylaminopropan, 3-Amino-1-cyclohexylaminopropan, 3-Amino-1- Methylaminobutan, Alkanolamine wie N-Aminoethylethanolamin, Ethanolamin, 3-Aminopropanol, Neopentanolamin.

Ferner können als Komponente b1) auch monofunktionelle Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin.

Bevorzugt werden 1,2-Ethylendiamin, Hydrazinhydrat, 1,4-Diaminobutan, Isophorondiamin und Diethylentriamin eingesetzt.

Unter ionisch bzw. potentiell ionisch hydrophilierenden Verbindungen der Komponente b2) werden sämtliche Verbindungen verstanden, die mindestens eine isocyanatreaktive Amino-Gruppe sowie mindestens eine Funktionalität, wie z.B. -COOY, -SO₃Y, -PO(OY)₂ (Y beispielsweise = H⁺, NH₄⁺, Metallkation), -SR₂, -NR₃⁺ (R = H, Alkyl, Aryl), aufweisen, die bei Wechselwirkung mit wässrigen Medien ein pH-Wert-abhängiges Dissoziationsgleichgewicht eingeht und auf diese Weise positiv, negativ oder neutral geladen sein kann.

Geeignete ionisch oder potentiell ionisch hydrophilierende Verbindungen sind zum Beispiel Mono- und Diaminocarbonsäuren, Mono- und Diaminosulfonsäuren sowie Mono- und Diaminophosphonsäuren und ihre Salze. Beispiele solcher ionischen bzw. potentiell ionischen Hydrophilierungsmittel sind N-(2-Anünoethyl)-ß-alanin, 2-(2-Amino-ethylamino)-ethansulfonsäure, Ethylendiamin-propyl- oder -butylsulfonsäure, 1,2- oder 1,3-Propylendiamin-ß-ethylsulfonsäure, Glycin, Alanin, Taurin, Lysin, 3,5-Diaminobenzoesäure und das Additionsprodukt von IPDI und Acrylsäure (EP-A 0 916 647, Beispiel 1). Weiterhin kann Cyclohexylaminopropansulfonsäure (CAPS) aus WO-A 01/88006 als anionisches oder potentiell anionisches Hydrophilierungsmittel verwendet werden.

Bevorzugte ionische oder potentiell ionische Hydrophilierungsmittel der Komponente b2) sind solche der vorstehend genannten Art, die über anionische, bevorzugt Carboxy- oder Carboxylat- und/oder Sulfonatgruppen hydrophilierend wirken.

Besonders bevorzugte ionische oder potentiell ionische Hydrophilierungsmittel b2) sind solche, die Carboxyl- und/oder Sulfonatgruppen als anionische oder potentiell anionische Gruppen enthalten, wie die Salze von N-(2-Aminoethyl)-ß-alanin, der 2-(2-Amino-ethylamino-)ethansulfonsäure oder des Additionsproduktes von IPDI und Acrylsäure (EP-A 0 916 647, Beispiel 1).

Zur Hydrophilierung wird bevorzugt eine Mischung aus anionischen bzw. potentiell anionischen Hydrophilierungsmitteln und nichtionischen Hydrophilierungsmitteln verwendet.

Das Verhältnis von NCO-Gruppen der Verbindungen aus Komponente a1) zu NCO-reaktiven Gruppen der Komponenten a2) bis a4) beträgt bei der Herstellung des NCO-funktionellen Prepolymers 1,05 bis 3,5, bevorzugt 1,2 bis 3,0 besonders bevorzugt 1,3 bis 2,5.

Die aminofunktionellen Verbindungen in Stufe B) werden in solch einer Menge eingesetzt, dass das Äquivalentverhältnis von isocyanatreaktiven Aminogruppen dieser Verbindungen zu den freien Isocyanatgruppen des Prepolymers 40 bis 150 %, bevorzugt zwischen 50 bis 125 %, besonders bevorzugt zwischen 60 bis 120 % beträgt.

In einer bevorzugten Ausführungsform werden anionisch und nichtionisch hydrophilierte Polyurethandispersionen eingesetzt, wobei zu deren Herstellung die Komponenten a1) bis a4) und b1) bis b2) in den folgenden Mengen eingesetzt werden, wobei sich die Einzelmengen zu 100 Gew.-% aufaddieren:
5 bis 40 Gew.-% Komponente a1),
55 bis 90 Gew.-% a2),
0,5 bis 20 Gew.-% Summe der Komponenten a3) und b1)
0,1 bis 25 Gew.-% Summe der Komponenten Komponente a4) und b2), wobei bezogen auf die Gesamtmengen der Komponenten a1) bis a4) und b1) bis b2) 0,1 bis 5 Gew.-% an anionischen oder potentiell anionischen Hydrophilierungsmitteln a4) und b2) verwendet werden.

Besonders bevorzugt betragen die Mengen der Komponente a1) bis a4) und b1) und b2):
5 bis 35 Gew.-% Komponente a1),
60 bis 90 Gew.-% a2),
0,5 bis 15 Gew.-% Summe der Komponenten a3) und b1)
0,1 bis 15 Gew.-% Summe der Komponenten Komponente a4) und b2), wobei bezogen auf die Gesamtmengen der Komponenten a1) bis a4) und b1) bis b2) 0,2 bis 4 Gew.-% an anionischen oder potentiell anionischen Hydrophilierungsmitteln a4) und b2) verwendet werden.

Ganz besonders bevorzugt betragen die Mengen der Komponente a1) bis a4) und b1) und b2):
10 bis 30 Gew.-% Komponente a1),
65 bis 85 Gew.-% a2),
0,5 bis 14 Gew.-% Summe der Komponenten a3) und b1)
0,1 bis 13,5 Gew.-% Summe der Komponenten a4) und b2), wobei bezogen auf die Gesamtmengen der Komponenten a1) bis a4) 0,5 bis 3,0 Gew.-% an anionischen oder potentiell anionischen Hydrophilierungsmitteln verwendet werden.

Besonders bevorzugte Ausführungsformen der Polyurethan Dispersionen (I) enthalten als Komponente als Komponente a1) Isophorondiisocyanat und/oder 1,6-Hexamthylendiisocyanat und/oder die isomeren Bis-(4,4'-isocyanatocyclohexyl)methane in Kombination mit a2) einer Mischung aus Polycarbonatpolyolen und Polytetramethylenglykolpolyolen.

Der Anteil der Polycarbonatpolyole in der Mischung a2) beträgt 20 bis 80 Gew.-% und 80 bis 20 Gew.-% an Polytetramethylenglykolpolyolen. Bevorzugt ist ein Anteil von 30 bis 75 Gew.-% an Polytetramethylenglykolpolyolen und 25 bis 70 Gew.-% an Polycarbonatpolyolen. Besonders bevorzugt ist ein Anteil von 35 bis 70 Gew.-% an Polytetramethylenglykolpolyolen und 30 bis 65 Gew.-% an Polycarbonatpolyolen, jeweils mit der Maßgabe, dass die Summe der Gewichtsprozente der Polycarbonat- und Polytetramethylenglykolpolyole 100 Gew.-% ergibt und der Anteil der Summe der Polycarbonat- und Polytetramethylenglykolpolyetherpolyole an der Komponente a2) mindestens 50 Gew.-% bevorzugt 60 Gew.-% und besonders bevorzugt mindestens 70 Gew.-% beträgt.

Die Herstellung solcher Polyurethandispersionen kann in einer oder mehreren Stufe/-n in homogener oder bei mehrstufiger Umsetzung, teilweise in disperser Phase durchgeführt werden. Nach vollständig oder teilweise durchgeführter Polyaddition aus a1) bis a4) erfolgt ein Dispergier-, Emulgier- oder Lösungsschritt. Im Anschluss erfolgt gegebenenfalls eine weitere Polyaddition oder Modifikation in disperser Phase.

Dabei können alle aus dem Stand der Technik bekannten Verfahren wie z. B. Prepolymer-Mischverfahren, Acetonverfahren oder Schmelzdispergierverfahren verwendet werden. Bevorzugt wird nach dem Aceton-Verfahren verfahren.

Für die Herstellung nach dem Aceton-Verfahren werden üblicherweise die Bestandteile a2) bis a4), die keine primären oder sekundären Aminogruppen aufweisen dürfen und die Polyisocyanatkomponente a1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon.

Andere Lösemittel (Colösemittel) wie Xylol, Toluol, Cyclohexan, Butylacetat, Methoxypropylacetat, N-Methylpyrrolidon, N-Ethylpyrrolidon, Lösemittel mit Ether- oder Estereinheiten können zusätzlich eingesetzt und ganz oder teilweise abdestilliert werden oder vollständig im Falle von, N-Methylpyrrolidon, N-Ethylpyrrolidon in der Dispersion verbleiben.

In einer besonderen Ausführungsform der Erfindung wird auf Colösemittel vollständig verzichtet.

Anschließend werden die gegebenenfalls zu Beginn der Reaktion noch nicht zugegebenen Bestandteile von a1) bis a4) zudosiert.

Die Umsetzung der Komponenten a1) bis a4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, z.B. Trialkylamine mit 1 bis 12, bevorzugt 1 bis 6 C-Atomen in jedem Alkylrest oder Alkalimetallbasen wie die entsprechenden Hydroxide eingesetzt.

Beispiele hierfür sind Trimethylamin, Triethylamin, Methyldiethylamin, Tripropylamin, N-methylmorpholin, Methyldiisopropylamin, Ethyldiisopropylamin und Diisopropylethylamin. Die Alkylreste können beispielsweise auch Hydroxylgruppen tragen, wie bei den Dialkylmonoalkanol-, Alkyldialkanol- und Trialkanolaminen. Als Neutralisationsmittel sind gegebenenfalls auch anorganische Basen, wie wässrige Ammoniaklösung oder Natrium- bzw. Kaliumhydroxid einsetzbar.

Bevorzugt sind Ammoniak, Triethylamin, Triethanolamin, Dimethylethanolamin oder Diisopropylethylamin sowie Natriumhydroxid.

Im Falle kationischer Gruppen werden Schwefelsäuredimethylester oder Bernsteinsäure oder Phosphorsäure eingesetzt.

Die Stoffmenge der Basen beträgt 50 und 125 mol-%, bevorzugt zwischen 70 und 100 mol-% der Stoffmenge der zu neutralisierenden Säuregruppen. Die Neutralisation kann auch gleichzeitig mit der Dispergierung erfolgen, in dem das Dispergierwasser bereits das Neutralisationsmittel enthält.

Im Anschluss wird in einem weiteren Verfahrensschritt, falls noch nicht oder nur teilweise geschehen das erhaltene Prepolymer mit Hilfe von aliphatischen Ketonen wie Aceton oder 2-Butanon gelöst.

Die aminischen Komponenten b1), b2), können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mitverwendet werden, so beträgt der Verdünnungsmittelgehalt in der in b) eingesetzten Komponente zur Kettenverlängerung bevorzugt 70 bis 95 Gew.-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Restgehalt an organischen Lösemitteln in den erfindungswesentlichen Dispersionen beträgt typischerweise weniger als 1,0 Gew.-%, bevorzugt weniger als 0,5 Gew.-%, besonders bevorzugt weniger als 0,1 Gew.-% und ganz besonders bevorzugt weniger als 0,05 Gew.-%bezogen auf die gesamte Dispersion.

Der pH-Wert der erfindungswesentlichen Dispersionen beträgt typischerweise weniger als 9,0, bevorzugt weniger als 8,5, besonders bevorzugt weniger als 8,0.

Der Feststoffgehalt der Polyurethandispersion beträgt typischerweise 20 bis 70 Gew.-%, bevorzugt 30 bis 65 Gew.-%, besonders bevorzugt 40 bis 63 Gew.-% und ganz besonders bevorzugt von 50 bis 63 Gew.-%.

Weiterhin ist es möglich, die erfindungswesentlichen Polyurethan-Polyharnstoff Dispersionen (I) durch Polyacrylate zu modifizieren. Hierzu wird in Gegenwart der Polyurethan-Dispersion eine Emulsionspolymerisation von olefinisch ungesättigten Monomeren, z. B. Estern aus (Meth)acrylsäure und Alkoholen mit 1 bis 18 C-Atomen, Styrol, Vinylestern oder Butadien durchgeführt, wie es z.B. in der DE-A-1 953 348, EP-A-0 167 188, EP-A-0 189 945 und EP-A-0 308 115 beschrieben ist. Die Monomere enthalten eine oder mehrere olefinische Doppelbindungen. Daneben können die Monomere funktionelle Gruppen wie Hydroxyl-, Epoxy-, Methylol- oder Acetoacetoxygruppen enthalten.

In einer besonderen bevorzugten Ausführungsform der Erfindung wird auf diese Modifizierung verzichtet.

Grundsätzlich ist es möglich, die erfindungswesentlichen Polyurethan-Polyharnstoff Dispersionen (I) mit anderen wässrigen Bindemitteln zu mischen. Solche wässrigen Bindemittel können z. B. aus Polyester-, Polyacrylat, Polyepoxid- oder Polyurethanpolymeren aufgebaut sein. Auch die Kombination mit strahlenhärtbaren Bindemitteln, wie sie z. B. in der EP-A-0 753 531 beschrieben sind, ist möglich. Es ist ebenfalls möglich die Polyurethan-Polyharnstoff Dispersionen (I) mit anderen anionischen oder nicht-ionischen Dispersionen, wie z.B. Polyvinylacetat, Polyethylen-, Polystyrol-, Polybutadien-, Polyvinylchlorid-, Polyacrylat- und Copolymerisat-Dispersionen, zu verschneiden.

In einer besonderen bevorzugten Ausführungsform der Erfindung wird auf diese Modifizierung verzichtet.

### Beispiele:

Sofern nicht abweichend gekennzeichnet, beziehen sich alle Prozentangaben auf das Gewicht.

Die Bestimmung der Festkörpergehalte erfolgte nach DIN-EN ISO 3251.

NCO-Gehalte wurden, wenn nicht ausdrücklich anders erwähnt, volumetrisch gemäß DIN-EN ISO 11909 bestimmt.

### Verwendete Substanzen und Abkürzungen:

- Diaminosulfonat:: NH₂-CH₂CH₂-NH-CH₂CH₂-SO₃Na (45 %ig in Wasser)
- Desmophen^{®} C2200:: Polycarbonatpolyol, OH-Zahl 56 mg KOH/g, zahlenmittleres Molekular- gewicht 2000 g/mol (Bayer Materialscience AG, Leverkusen, DE)
- PolyTHF^{®} 2000:: Polytetramethylenglykolpolyol, OH-Zahl 56 mg KOH/g, zahlenmittleres Molekulargewicht 2000 g/mol (BASF AG, Ludwigshafen, DE)
- PolyTHF^{®} 1000:: Polytetramethylenglykolpolyol, OH-Zahl 112 mg KOH/g, zahlen- mittleres zahlenmittleres Molekulargewicht 1000 g/mol (BASF AG, Ludwigshafen, DE)
- Polyether LB 25:: (monofunktioneller Polyether auf Ethylenoxid-/Propylenoxidbasis zahlenmittleres Molekulargewicht 2250 g/mol, OH-Zahl 25 mg KOH/g (Bayer Materialscience AG, Leverkusen, DE)
- Stokal^{®} STA:: Schaumhilfsmittel auf Ammoniumstearat-Basis, Wirkstoffgehalt: 30 % (Bozzetto GmbH, Krefeld, DE)
- Stokal^{®} SR:: Schaumhilfsmittel auf Succinamat-Basis, Wirkstoffgehalt: ca. 34 % (Bozzetto GmbH, Krefeld, DE)
- Mirox AM:: wässrige Acrylsäure-Copolymer Dispersion (BGB Stockhausen GmbH, Krefeld, DE)
- Borchigel ALA:: wässrige, anionische Acrylpolymer-Lösung (Borchers GmbH, Langenfeld, DE)
- Octosol SLS:: wässrige Natriumlaurylsulfat-Lösung (Tiarco Chemical Europe GmbH, Nürnberg, DE)
- Octosol 845.: Natriumlaurylsulfatether (Tiarco Chemical Europe GmbH, Nürnberg, DE)

Die Bestimmung der mittleren Teilchengrößen (angegeben ist das Zahlenmittel) der PUR-Dispersionen erfolgte mittels Laserkorrelations-Spektroskopie (Gerät: Malvern Zetasizer 1000, Malver Inst. Limited).

### Beispiel 1: PUR-Dispersion (Komponente 1)

144,5 g Desmophen^{®} C2200, 188,3 g PolyTHF^{®} 2000, 71,3 g PolyTHF^{®} 1000 und 13,5 g Polyether LB 25 wurden auf 70°C aufgeheizt. Anschließend wurde bei 70°C innerhalb von 5 min ein Gemisch aus 45,2 g Hexamethylendiisocyanat und 59,8 g Isophorondiisocyanat zugegeben und solange unter Rückfluss gerührt bis der theoretische NCO-Wert erreicht war. Das fertige Prepolymer wurde mit 1040 g Aceton bei 50°C gelöst und anschließend eine Lösung aus 1,8 g Hydrazinhydrat, 9,18 g Diaminosulfonat und 41,9 g Wasser innerhalb von 10 min zudosiert. Die Nachrührzeit betrug 10 min. Nach Zugabe einer Lösung aus 21,3 g Isophorondiamin und 106,8 g Wasser wurde innerhalb von 10 min durch Zugabe von 254 g Wasser dispergiert. Es folgte die Entfernung des Lösemittels durch Destillation im Vakuum und es wurde eine lagerstabile Dispersion mit einem Festkörpergehalt von 60,0 % erhalten.

### Beispiel 2: PUR-Dispersion (Komponente I)

2159,6 g eines difunktionellen Polyesterpolyols auf Basis Adipinsäure, Neopentylglykol und Hexandiol (mittleres Molgekulargewicht 1700 g/mol, OHZ = 66), 72,9 g eines monofunktionellen Polyethers auf Ethylenoxid-/Propylenoxidbasis (70/30) (mittlerem Molekulargewicht 2250 g/mol, OH-Zahl 25 mg KOH/g) wurden auf 65°C aufgeheizt. Anschließend wurde bei 65°C innerhalb von 5 min ein Gemisch aus 241,8 g Hexamethylendiisocyanat und 320,1 g Isophorondiisocyanat zugegeben und solange bei 100°C gerührt bis der theoretische NCO-Wert von 4,79 % erreicht wurde. Das fertige Prepolymer wurde mit 4990 g Aceton bei 50°C gelöst und anschließend eine Lösung aus 187,1 g Isophorondiamin und 322,7 g Aceton innerhalb von 2 min zudosiert. Die Nachrührzeit betrug 5 min. Anschließend wurde innerhalb von 5 min eine Lösung aus 63,6 g Diaminosulfonat, 6,5 Hydrazinhydrat und 331,7 g Wasser zudosiert. Die Dispergierung erfolgte durch Zugabe von 1640,4 g Wasser. Es folgte die Entfernung des Lösemittels durch Destillation im Vakuum und es wurde eine lagerstabile PUR-Dispersion mit einem Festkörpergehalt von 58,9 % erhalten.

### Beispiel 3: PUR-Dispersion (Komponente I)

2210,0 g eines difunktionellen Polyesterpolyols auf Basis Adipinsäure, Neopentylglykol und Hexandiol (mittleres Molgekulargewicht 1700 g/mol, OHZ = 66) wurde auf 65°C aufgeheizt. Anschließend wurde bei 65°C innerhalb von 5 min ein Gemisch aus 195,5 g Hexamethylendiisocyanat und 258,3 g Isophorondiisocyanat zugegeben und solange bei 100°C gerührt bis der theoretische NCO-Wert von 3,24% erreicht wurde. Das fertige Prepolymer wurde mit 4800 g Aceton bei 50°C gelöst und anschließend eine Lösung aus 29,7 g Ethylendiamin, 95,7 g Diaminosulfonat und 602 g Wasser innerhalb von 5 min zudosiert. Die Nachrührzeit betrug 15 min. Anschließend wurde innerhalb von 20 min durch Zugabe von 1169 g Wasser dispergiert. Es folgte die Entfernung des Lösemittels durch Destillation im Vakuum und es wurde eine lagerstabile PUR-Dispersion mit einem Festkörpergehalt von 60 % erhalten.

### Beispiel 4: Herstellung einer Kaumasse

1000 g einer kommerziell verfügbaren Polyurethandispersion (I) (Impranil DLU, Bayer MaterialScience AG, Deutschland) wurden mit 15 g Stokal STA (II), 20 g Stokal SR (II) und 30 g Borchigel ALA (IV) gemischt und anschließend durch Eintragen von Luft mit Hilfe eines Handmixgerätes aufgeschäumt. Die erhaltene Schaumdichte betrug 400 g/l. Danach wurde die geschäumte Paste mit einem Filmziehgerät, bestehend aus zwei polierten Walzen, die auf einen exakten Abstand eingestellt werden können und wobei vor die hintere Walze ein Trennpapier eingelegt wurde, aufgebracht. Mit einer Fühlerblattlehre wurde der Abstand zwischen Papier und vorderer Walze eingestellt. Dieser Abstand entsprach der Filmdicke (nass) der resultierenden Beschichtung, die so gewählt wurde, dass eine Trockenschichtdicke > 100 µm erzielt wurde. Abschließend wurde das Material in einem Trockenschrank bei 80°C 15 Minuten getrocknet. Nach Abzug des Trennpapieres erhielt man die erfindungsgemäße Kaumasse. Die anwendungstechnischen Eigenschaften sind in Tabelle 1 wiedergegeben.

### Beispiel 5: Herstellung einer Kaumasse

1000 g der aus Beipiel 1 erhaltenen Dispersion (I) wurden mit 30 g Octosol SLS (II), 20 g Stokal SR (II), 20 g Octosol 845 (II), 5 g 5%iger Ammoniak-Lösung und 15g Mirox AM (IV) gemischt und anschließend durch Eintragen von Luft mit Hilfe eines Handmixgerätes aufgeschäumt. Die erhaltene Schaumdichte betrug 400 g/l. Danach wurde die geschäumte Paste mit einem Filmziehgerät, bestehend aus zwei polierten Walzen, die auf einen exakten Abstand eingestellt werden können und wobei vor die hintere Walze ein Trennpapier eingelegt wurde, aufgebracht. Mit einer Fühlerblattlehre wurde der Abstand zwischen Papier und vorderer Walze eingestellt. Dieser Abstand entsprach der Filmdicke (nass) der resultierenden Beschichtung, die so gewählt wurde, dass eine Trockenschichtdicke > 100 µm erzielt wurde. Abschließend wurde das Material in einem Trockenschrank bei 80°C 15 Minuten getrocknet. Nach Abzug des Trennpapieres erhielt man die erfindungsgemäße Kaumasse. Die anwendungstechnischen Eigenschaften sind in Tabelle 1 wiedergegeben.

**Tabelle 1: Anwendungstechnische Eigenschaften der erfindungsgemäßen Kaumassen**

| Kaumasse aus: | 100%v Modul [MPa] | Dehnung [%] | Zugfestigkeit [MPa] |
|---|---|---|---|
| Beispiel 4 | 0,6 | 570 | 2,4 |
| Beispiel 5 | 0,8 | 710 | 5,2 |

Die Bestimmung des Moduls bei 100 % Dehnung erfolgte an Filmen mit einer Schichtdicke > 100 µm.

| Kaumasse aus: | α_{f}/E | R x E /σ_{f}² |
|---|---|---|
| Beispiel 4 | 1,4 | 1,5 |
| Beispiel 5 | 1,6 | 1,3 |

| | | |
|---|---|---|
| σ_{f}: Zugfestigkeit E: Elastizitätsmodul R: Weiterreißfestigkeit | | |

## Patentansprüche

1. Verwendung von geschäumten synthetischen Polymeren als Kaumassen, **dadurch gekennzeichnet, dass** die Kaumassen auf wässrigen Polyurethan-Dispersionen basieren.

2. Verwendung gemäß Anspruch 1, **dadurch gekennzeichnet, dass** die Kaumassen nicht plastisch verformbar sind.

3. Verwendung gemäß einem der Ansprüche 1 oder 2, **dadurch gekennzeichnet, dass** die Kaumassen ein Zugmodul bei 100% Dehnung von 0,3 bis 3,5 MPa, bei einer Zugfestigkeit von 0,5 bis 40 MPa und einer Dehnbarkeit von 200 bis 2000 % aufweisen.

4. Verwendung gemäß einem der Ansprüche 1 bis 3, **dadurch gekennzeichnet, dass** die Kaumassen ein Verhältnis von Zugfestigkeit zu Elastizitätsmodul von größer gleich 1 und ein Verhältnis aus dem Produkt der Weiterreissfestigkeit (gemäß DIN ISO 34-1 (2004)) und Elastizitätsmodul zum Quadrat der Zugfestigkeit kleiner als 4 mm aufweisen.

5. Verwendung gemäß einem der Ansprüche 1 bis 4, **dadurch gekennzeichnet, dass** die Dispersionen erhältlich sind, indem isocyanatfunktionelle Prepolymere aus
a1) organischen Polyisocyanaten
a2) polymeren Polyolen mit zahlenmittleren Molekulargewichten von 400 bis 8000 g/mol und OH-Funktionalitäten von 1,5 bis 6,
a3) gegebenenfalls hydroxyfunktionellen Verbindungen mit Molekulargewichten von 62 bis 399 g/mol und
a4) gegebenenfalls hydroxyfunktionellen, ionischen oder potentiell ionischen und/oder nichtionischen Hydrophilierungsmitteln,
hergestellt werden,
deren freie NCO-Gruppen dann ganz oder teilweise mit
b1) aminofunktionellen Verbindungen mit Molekulargewichten von 32 bis 400 g/mol und/oder
b2) aminofunktionellen, ionischen oder potentiell ionischen Hydrophilierungsmitteln
unter Kettenverlängerung umgesetzt werden und die Prepolymere vor während oder nach Schritt B) in Wasser dispergiert werden, wobei gegebenenfalls enthaltene potentiell ionische Gruppen durch teilweise oder vollständige Umsetzung mit einem Neutralisationsmittel in die ionische Form überführt werden können.

6. Verwendung gemäß einem der Ansprüche 1 bis 5 für den Mundpflegebereich.

## Claims

1. Use of foamed synthetic polymers as masticatory compositions, **characterized in that** the masticatory compositions are based on aqueous polyurethane dispersions.

2. Use according to claim 1, **characterized in that** the masticatory compositions are not plastically deformable.

3. Use according to one of claims 1 or 2, **characterized in that** the masticatory compositions have a tensile modulus at 100 % elongation of from 0.3 to 3.5 MPa at a tensile strength of from 0.5 to 40 MPa and an extensibility of from 200 to 2,000 %.

4. Use according to one of claims 1 to 3, **characterized in that** the masticatory compositions have a ratio of tensile strength to elasticity modulus of greater than equal to 1 and a ratio of the product of the tear propagation resistance (in accordance with DIN ISO 34-1 (2004)) and elasticity modulus to the square of the tensile strength of less than 4 mm.

5. Use according to one of claims 1 to 4, **characterized in that** the dispersions are obtainable by a procedure in which isocyanate-functional prepolymers are prepared from
a1) organic polyisocyanates
a2) polymeric polyols having number-average molecular weights of from 400 to 8,000 g/mol and OH functionalities of from 1.5 to 6
a3) optionally hydroxy-functional compounds having molecular weights of from 62 to 399 g/mol and
a4) optionally hydroxy-functional, ionic or potentially ionic and/or nonionic hydrophilizing agents,
the free NCO groups thereof are then reacted completely or partially with
b1) amino-functional compounds having molecular weights of from 32 to 400g/mol and/or
b2) amino-functional, ionic or potentially ionic hydrophilizing
agents
with chain lengthening and the prepolymers are dispersed in water before, during or after step B), it being possible, where appropriate, for potentially ionic groups present to be converted into the ionic form by partial or complete reaction with a neutralizing agent.

6. Use according to one of claims 1 to 5 for the oral care sector.

## Revendications

1. Utilisation de polymères synthétiques expansés en tant que gommes à mâcher, **caractérisée en ce que** les gommes à mâcher sont à base de dispersions aqueuses de polyuréthanne.

2. Utilisation selon la revendication 1, **caractérisée en ce que** les gommes à mâcher ne peuvent subir de déformation plastique.

3. Utilisation selon l'une des revendications 1 ou 2, **caractérisée en ce que** les gommes à mâcher présentent un module d'élasticité en traction pour un allongement de 100 % de 0,3 à 3,5 MPa, pour une résistance à la traction de 0,5 à 40 MPa et une extensibilité de 200 à 2000 %.

4. Utilisation selon l'une des revendications 1 à 3, **caractérisée en ce que** les gommes à mâcher présentent un rapport de leur résistance à la traction à leur module d'élasticité supérieure ou égale à 1 et un rapport entre le produit de la résistance à la déchirure amorcée (selon DIN ISO 34-1 (2004)) par le module d'élasticité, et le carré de la résistance à la traction, inférieur à 4 mm.

5. Utilisation selon l'une des revendications 1 à 4, **caractérisée en ce qu'**on peut préparer les dispersions en préparant des prépolymères à fonctionnalité isocyanate à partir
a1) de polyisocyanates organiques,
a2) de polyols polymères ayant une masse moléculaire moyenne en nombre de 400 à 8000 g/mole et une fonctionnalité OH de 1,5 à 6,
a3) éventuellement, de composés à fonctionnalité hydroxy, ayant une masse moléculaire de 62 à 399 g/mole, et
a4) éventuellement, d'agents d'hydrophilisation à fonctionnalité hydroxy, ioniques ou potentiellement ioniques et/ou non ioniques,
dont les groupes NCO libres sont ensuite mis à réagir en totalité ou en partie avec
b1) des composés à fonctionnalité amino, ayant une masse moléculaire de 32 à 400 g/mole, et/ou
b2) des agents d'hydrophilisation à fonctionnalité amino, ioniques ou potentiellement ioniques,
en présence d'une extension de chaîne, et on disperse les polymères dans de l'eau avant, pendant ou après l'étape B), ce à l'occasion de quoi les groupes potentiellement ioniques éventuellement présents peuvent, par une réaction partielle ou complète, être convertis en leur forme ionique à l'aide d'un agent de neutralisation.

6. Utilisation selon l'une des revendications 1 à 5 dans le domaine des soins buccaux.
